# EUROPEAN PATENT APPLICATION

(11) **EP 4 534 018 A1**
(43) Date of publication of application: **09.04.2025**
(21) Application number: 23816421.4
(22) Date of filing: 05.06.2023
(51) Int. Cl.: A61B 8/08, G06N 3/08, G16H 50/20

(54) **METHOD FOR PROVIDING INFORMATION REGARDING ULTRASOUND IMAGE VIEW AND DEVICE FOR PROVIDING INFORMATION REGARDING ULTRASOUND IMAGE VIEW BY USING SAME**

(30) Priority: 03.06.2022 KR 20220068536
(71) Applicant: Ontact Health Co., Ltd., Seoul 03764 (KR)
(72) Inventor: SHIM, Hack Joon, Seoul 06214 (KR); HA, Seong Min, Seoul 05343 (KR); JEONG, Hyun Seok, Seoul 03725 (KR); JEON, Jae Ik, Uijeongbu-si Gyeonggi-do 11742 (KR); CHOI, Annes, Seoul 04193 (KR)
(74) Representative: Müller Schupfner & Partner Patent- und Rechtsanwaltspartnerschaft mbB (Muc)
(86) International application number: PCT/KR2023/007715
(87) International publication number: WO 2023/234762

(57) **Abstract**

The present invention provides a method for providing information regarding an ultrasound image view, which is implemented by a processor, and a device using the method, the method comprising the steps of: receiving an ultrasound image of an object; and classifying respective image views on the basis of the received ultrasound image, by using an image view classification model trained to classify a plurality of image views by using an ultrasound image as an input and output respective image views, wherein the respective image views indicate ultrasound image views in a plurality of different ultrasound modes.

## Description

### [Technical Field]

The present invention relates to a method for providing information regarding ultrasound image view and a device for providing information regarding ultrasound image view by using the same.

### [Background Art]

Ultrasound examination is performed by projecting ultrasound waves on a three-dimensional object in multiple-dimensional planes to obtain images and measure hemodynamic variables.

For example, in the case of a cardiac ultrasound image, a medical staff places an ultrasound probe in a location where it is easy to obtain an ultrasound image and records images by finding appropriate slices by rotating and tilting the ultrasound probe, thereby obtaining multifaceted images from anatomical structures around the heart between the ribs.

In the meantime, in recent years, in accordance with the development of the technology, a technique which integrates a plurality of ultrasound elements to simultaneously obtain a lot of information and select desired information, among them, to see the information.

However, the image obtaining process is performed by the medical staff so that a long training period may be required to perform the appropriate examination.

In the meantime, the ultrasound examination process is divided into examination preparation, image acquisition, image analysis, and report writing, and specifically, it takes a lot of time for image acquisition and analysis processes.

At this time, when an image corresponding to a standard measurement image view is not acquired in the image analysis process, re-examination is inevitable and an error may occur in the essential measurement value and judgment of the examination time may be essentially required according to each image view.

That is, a deviation of the acquired ultrasound image may be large depending on a skill level of the medical staff so that there is a continuous demand for the development of a new information providing system which is capable of recognizing and classifying image views.

The background of the present invention is described for easier understanding of the present invention. It should not be understood to admit the matters described in the background of the present invention as a prior art.

### [Prior Art Document]

### [Non-patent Documents]

(Non-patent Document 1) Rahul C. Deo., et al. Circulation. 2018;138:1623-1635.; Fully Automated Echocardiogram Interpretation in Clinical Practice
(Non-patent Document 2) LASSE LOVSTAKKEN., et al. Ultrasound in Med. & Biol., Vol. 45, No. 2, pp. 374-384, 2019.; REAL-TIME STANDARD VIEW CLASSIFICATION IN TRANSTHORACIC ECHOCARDIOGRAPHY USING CONVOLUTIONAL NEURAL NETWORKS
(Non-patent Document 3) Bjørnar Grenne., et al. JACC: CARDIOVASCULAR IMAGING, VOL. 14, NO. 10, 2021 OCTOBER 2021:1918-1928.; Artificial Intelligence for Automatic Measurement of Left Ventricular Strain in Echocardiography
(Non-patent Document 4) Sultan Almotairi., et al. An Accurate and Fast Cardio-views Classification System Based on Fused Deep Features and LSTM

### [Detailed Description of the Invention]

### [Technical Problem]

In the meantime, in order to solve the above-mentioned problems, the inventors of the present invention have tried to develop an artificial neural network-based information providing system trained to recognize an image view for an ultrasound image and distinguish the ultrasound image for every image view.

Specifically, the inventors of the present invention have recognized that image views may be recognized and classified, regardless of an ultrasound mode, such as a B-mode, an M-mode, and a Doppler mode, in the ultrasound image by applying an artificial neural network.

To be more specific, the inventors of the present invention paid attention to an artificial neural network trained to classify corresponding image views using an ultrasound image as an input. As a result, the inventors of the present invention have recognized that the image view may be classified with a high accuracy without having a record for an examination time, for the ultrasound image having image views acquired in a plurality of modes.

In the meantime, the inventors of the present invention paid more attention to an ultrasound image having a digital imaging and communications in medicine (DICOM) format with various tagging information.

To be more specific, the inventors of the present invention paid attention that a variety of metadata indicating what the ultrasound mode of each image is, in the ultrasound image having a DICOM format and further whether the ultrasound image is a still-cut image or a moving image, a color image, or a monochromic image is utilized.

As a result, the inventors of the present invention have recognized that the ultrasound image for which the image view was classified based on the metadata is verified (or corrected) and highly reliable information regarding the image view is provided.

As a result, the inventors of the present invention have developed an information providing system for ultrasound image views which is capable of classifying image views of the ultrasound image on the basis of the artificial neural network and verifying image views on the basis of the metadata.

The inventors of the present invention have recognized that a new information providing system is provided to reduce unnecessary diagnosis time and acquire a standardized image view with an artificial neural network-based system.

That is, the inventors of the present invention have expected that a new information providing system is provided to acquire an ultrasound image regardless of the skill level of the medical staff and provide a highly reliable analysis result for the ultrasound image.

Therefore, an object to be achieved by the present invention is to provide a method for providing information regarding an ultrasound image view configured to recognize and classify respective image views from a received ultrasound image using an artificial neural network-based classification model and a device using the same.

Therefore, another object to be achieved by the present invention is to provide a method for providing information regarding an ultrasound image view configured to verify image views for each classified ultrasound image, using metadata of the ultrasound images, and a device using the same.

Objects of the present invention are not limited to the above-mentioned objects, and other objects, which are not mentioned above, can be clearly understood by those skilled in the art from the following descriptions.

### [Means for Solving the Problem]

In order to achieve the above-described objects, according to an aspect of the present invention, a method for providing information regarding an ultrasound image view is provided. The method for providing information includes:

receiving an ultrasound image of an object; and classifying respective image views on the basis of the received ultrasound image, by using an image view classification model trained to classify a plurality of image views by using an ultrasound image as an input and output respective image views, where the respective image views indicate ultrasound image views in a plurality of different ultrasound modes.

According to a feature of the present invention, the plurality of ultrasound modes includes an M-mode and a B-mode and the ultrasound image is an echocardiographic image, and the step of classifying respective image views may include a step of determining at least one M-mode image view, among an M-mode (PLAX/PSAX) through a great artery, an M-mode (PLAX/PSAX) through MV, and an M-mode (PLAX/PSAX) through LV, and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the ultrasound image, using the image view classification model.

According to another feature of the present invention, the plurality of ultrasound modes includes a Doppler mode and a B-mode and the ultrasound image is an echocardiographic image, and the step of classifying respective image views may include a step of determining at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the ultrasound image, using the image view classification model.

According to still another feature of the present invention, the plurality of ultrasound modes includes a Doppler mode, an M-mode, and a B-mode and the ultrasound image is an echocardiographic image, and the step of classifying respective image views may include a step of determining at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), at least one M-mode image view, among an M-mode (PLAX/PSAX) through a great artery, an M-mode (PLAX/PSAX) through MV, and an M-mode (PLAX/PSAX) through LV, and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the ultrasound image, using the image view classification model.

According to still another feature of the present invention, the plurality of ultrasound modes may include at least one of an M-mode, a Doppler mode, and a B-mode and a non-classified mode defined as an ultrasound image view which is different from the M-mode, the Doppler mode, and the B-mode.

According to still another feature of the present invention, the ultrasound image is an echocardiographic image and the step of classifying respective image views may include a step of classifying 4-chamber, 2-chamber, long axis, base, mid, and apex image views for the ultrasound image, using the image view classification model.

According to still another feature of the present invention, the method for providing information may further include: after the step of classifying respective image views, a step of verifying respective image views.

According to still another feature of the present invention, the step of verifying respective image views may include a step of determining whether the ultrasound image is a still cut image or a moving image on the basis of the number of frames of respective image views.

According to still another feature of the present invention, the step of verifying respective image views may include a step of determining whether the respective image views are color images or monochromic images.

According to still another feature of the present invention, the ultrasound image is an image of digital imaging and communications in medicine (DICOM) format displaying metadata including tagging information regarding an ultrasound mode of the ultrasound image. At this time, the step of verifying respective image views may include a step of verifying the respective classified views on the basis of the metadata, and after the step of verifying, may further include a step of correcting a classification result of the respective image views when the respective image views are different from tagging information.

According to still another feature of the present invention, the image view classification model may have output nodes corresponding to the number of the plurality of image views according to the plurality of ultrasound modes.

In order to achieve the above-described objects, according to another exemplary embodiment of the present invention, a device for providing information regarding an ultrasound image view is provided. At this time, the device for providing information includes a communication unit configured to receive an ultrasound image of an object; and a processor operably connected to the communication unit. At this time, the processor is configured to classify respective image views on the basis of the received ultrasound image, by using an image view classification model trained to classify a plurality of image views by using an ultrasound image as an input and output respective image views, and the respective image views indicate ultrasound image views in a plurality of different ultrasound modes.

According to a feature of the present invention, the ultrasound image is an echocardiographic image and the processor may be configured to determine at least one M-mode image view, among an M-mode (PLAX/PSAX) through a great artery, an M-mode (PLAX/PSAX) through MV, and an M-mode (PLAX/PSAX) through LV, and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the ultrasound image, using the image view classification model.

According to another feature of the present invention, the ultrasound image is an echocardiographic image and the processor may be configured to determine at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the ultrasound image, using the image view classification model.

According to still another feature of the present invention, the ultrasound image is an echocardiographic image and the processor may be configured to determine at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), at least one M-mode image view, among an M-mode (PLAX/PSAX) through a great artery, an M-mode (PLAX/PSAX) through MV, and an M-mode (PLAX/PSAX) through LV, and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the ultrasound image, using the image view classification model.

According to still another feature of the present invention, the ultrasound image is an echocardiographic image and the processor may be configured to classify 4-chamber, 2-chamber, long axis, base, mid, and apex image views for the ultrasound image, using the image view classification model.

According to still another feature of the present invention, the processor may be further configured to verify the respective image views.

According to still another feature of the present invention, the processor may be further configured to determine whether the ultrasound image is a still cut image or a moving image on the basis of the number of frames of respective image views.

According to still another feature of the present invention, the processor may be further configured to determine whether the respective image views are color images or monochromic images.

According to still another feature of the present invention, the processor may be further configured to verify the respective image views on the basis of the metadata and correct a classification result of the respective image views when the respective classified image views are different from the tagging information.

Other detailed matters of the exemplary embodiments are included in the detailed description and the drawings.

### [Effect of the Invention]

According to the present invention, an information providing system for an ultrasound image view based on the artificial neural network configured to classify the ultrasound image views using the ultrasound image is provided, thereby providing highly reliable echocardiographic diagnosis result.

Further, according to the present invention, an image providing system which recognizes, classifies and verifies respective image views regardless of the set ultrasound mode is provided to be generally applied to various ultrasound analysis systems which provide an ultrasound image with a DICOM format.

Moreover, according to the present invention, an image providing system which verifies (or corrects) an ultrasound image for which image views are classified on the basis of metadata, is provided to provide more reliable information regarding image views.

That is, according to the present invention, the information providing system for ultrasound image views on the basis of the artificial neural network is provided so that the ultrasound image may be acquired regardless of the skill level of the medical staff, thereby contributing to establishing more accurate decision-making and a treatment plan in the image analysis step.

The effects according to the present invention are not limited to the contents exemplified above, and more various effects are included in the present specification.

### [Brief Description of the Drawings]

FIG. 1 illustrates an information providing system for an ultrasound image view using a device for providing information regarding an ultrasound image view according to an exemplary embodiment of the present invention.
FIG. 2A is a block diagram illustrating a configuration of a medical staff device according to an exemplary embodiment of the present invention.
FIG. 2B is a block diagram illustrating a configuration of an information providing server according to an exemplary embodiment of the present invention.
FIG. 3 illustrates a procedure of a method for providing information regarding an ultrasound image view according to an exemplary embodiment of the present invention.
FIGS. 4A to 4E exemplarily illustrate a procedure of a method for providing information regarding an ultrasound image view according to an exemplary embodiment of the present invention.
FIGS. 5A and 5B illustrate a procedure of a method for providing information regarding an ultrasound image view according to another exemplary embodiment of the present invention.
FIG. 6 exemplarily illustrates a procedure of a method for providing information regarding an ultrasound image view according to another exemplary embodiment of the present invention.
FIG. 7 exemplarily illustrates a structure of an image view classification model which is used for an information providing method according to various exemplary embodiments of the present invention.
FIG. 8 exemplarily illustrates learning data of an image view classification model which is used for an information providing method according to various exemplary embodiments of the present invention.
FIG. 9 illustrates an evaluation result of an image view classification model which is used for an information providing method according to various exemplary embodiments.
FIG. 10 illustrates a procedure of a method for providing information regarding an ultrasound image view according to various exemplary embodiments of the present invention.
FIG. 11 illustrates a procedure of a method for providing information regarding an ultrasound image view according to another exemplary embodiment of the present invention.

### [Best Mode for Carrying Out the Invention]

Advantages of the present invention and a method of achieving the advantages and characteristics will be clear by referring to exemplary embodiments described below in detail together with the accompanying drawings. However, the present invention is not limited to the exemplary embodiment disclosed herein but will be implemented in various forms. The exemplary embodiments are provided by way of example only so that a person of ordinary skilled in the art can fully understand the disclosures of the present invention and the scope of the present invention. Therefore, the present invention will be defined by the scope of the appended claims.

The shapes, sizes, ratios, angles, numbers, and the like illustrated in the accompanying drawings for describing the exemplary embodiments of the present invention are merely examples, and the present invention is not limited thereto. Further, in the following description, a detailed explanation of known related technologies may be omitted to avoid unnecessarily obscuring the subject matter of the present invention. The terms such as "including," "having," and "consist of" used herein are generally intended to allow other components to be added unless the terms are used with the term "only". Any references to singular may include plural unless expressly stated otherwise.

Components are interpreted to include an ordinary error range even if not expressly stated.

The features of various exemplary embodiments of the present invention can be partially or entirely bonded to or combined with each other and can be interlocked and operated in technically various ways understood by those skilled in the art, and the exemplary embodiments can be carried out independently of or in association with each other.

For clarity of interpretation of the present specification, terms used in the present specification will be defined below.

The term used in the present specification, "object" may refer to all targets who want to be provided with information regarding an ultrasound image view. In the meantime, the object disclosed in the present specification may be any mammal other than a human, but is not limited thereto.

The term used in the present invention, "ultrasound image" is an ultrasound image which can be obtained by a non-invasive method.

In the preferred exemplary embodiment, the ultrasound image may be an echocardiographic image, but is not limited thereto.

In the meantime, the ultrasound image may be a still-cut image or a moving image configured by a plurality of cuts. For example, for the moving image, ultrasound image views for a frame of the moving image may be classified according to a method for providing information regarding an ultrasound image view according to an exemplary embodiment of the present invention. As a result, according to the present invention, the image views are classified as soon as the ultrasound image is received from an image diagnosis device to provide a streaming service and ultrasound image view information may be provided in real time.

In the meantime, the ultrasound image may be a 2D moving image, but is not limited thereto and may be a 3D image.

According to the feature of the present invention, the ultrasound image may be an image with a DICOM format including metadata.

Here, "metadata" may correspond to DICOM tag information.

At this time, the metadata may include information regarding an ultrasound image indicating what an ultrasound mode of each image is. However, it is not limited thereto and the metadata may further include information regarding whether the ultrasound image is a still-cut image, a moving image, a color image, or a monochromic image.

That is, according to various exemplary embodiments of the present invention, metadata may be used to verify and correct an image view classification result.

According to still another feature of the present invention, the ultrasound image may include a plurality of ultrasound image views in an M-mode, a B-mode, a Doppler mode, or other modes.

The term used in the present invention, "ultrasound image view" encompasses an image view according to a position of the ultrasound probe, that is, a capturing position, and further, an image corresponding to an image view.

For example, the ultrasound image is an echocardiographic image and may include 4-chamber, 2-chamber, long axis, base, mid, and apex ultrasound image views acquired in the M-mode, B-mode, Doppler mode, or other modes.

To be more specific, the image view may include at least one M-mode image view, among an M-mode (PLAX/PSAX) through a great artery, an M-mode (PLAX/PSAX) through MV, and an M-mode (PLAX/PSAX) through LV.

Moreover, the image view may include at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC).

Moreover, the image view may include at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C).

However, the present invention is not limited thereto and the image view may further include an image view in a non-classified mode (other modes).

In the meantime, from the ultrasound image including a plurality of ultrasound modes and/or a plurality of image views, ultrasound image views may be recognized and classified in the images by the image view classification model, regardless of the ultrasound mode.

The term used in the present invention, "image view classification model" may be a model configured to output an ultrasound image view using the ultrasound image as an input.

According to the feature of the present invention, the image view classification model may be a model trained to classify image views in the M-mode, B-mode, or Doppler mode, on the basis of the ultrasound image for training. At this time, the ultrasound image for training may be an image labeled with an image view in the M-mode, B-mode, or Doppler mode.

According to another feature of the present invention, the image view classification model may be a model having three output nodes trained to classify three image views of M-mode (PLAX//PSAX), M-mode (PLAX/PSAX) through MV, and M-mode (PLAX/PSAX) through LV for the M-mode ultrasound image.

According to still another feature of the present invention, the image view classification model may be a model having 17 output nodes trained to classify 17 image views of PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the B-mode ultrasound image.

According to still another feature of the present invention, the image view classification model may be a model having 14 output nodes trained to classify 14 image views of MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), for a Doppler mode ultrasound image.

However, the present invention is not limited thereto and the image view classification model may have five output nodes to classify five image views of non-classified modes, such as non-classified (B mode), non-classified (M mode), non-classified (Doppler mode), non-classified (color Doppler mode), and non-classified image views.

According to still another feature of the present invention, the image view classification model may be a model having 39 output nodes to classify all image views of three M-mode image views, 17 B-mode image views, 14 Doppler mode image views, and 5 non-classified image views using the ultrasound image as an input.

That is, according to various exemplary embodiments of the present invention, the image view classification model may classify image views with a high accuracy, regardless of the mode of the input image.

In the meantime, the image view classification model may be a DenseNet-121-based model, but is not limited thereto. For example, the prediction models may be based on at least one algorithm selected from a fully convolutional network (FCN) having U-net, VGG net, DenseNet, and an encoder-decoder structure, a deep neural network (DNN) such as SegNet, DeconvNet, and DeepLAB V3+, or SqueezeNet, Alexnet, ResNet18, MobileNet-v2, GoogLeNet, Resnet-v2, Resnet50, RetinaNet, Resnet101, and Inception-v3. Moreover, the image view classification model may be an ensemble model based on at least two algorithm models, among the above-described algorithms.

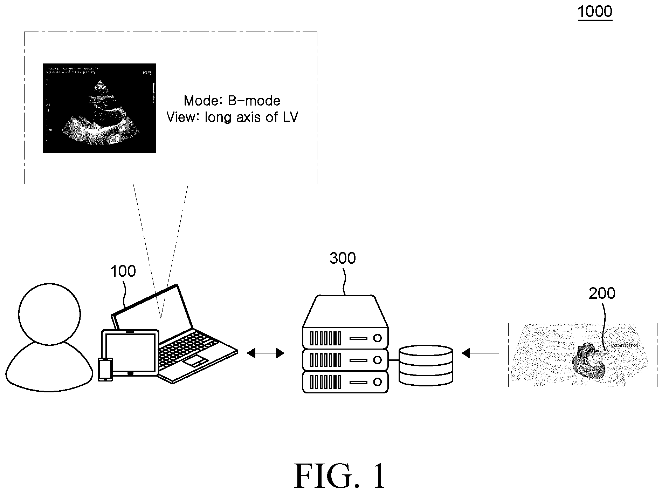

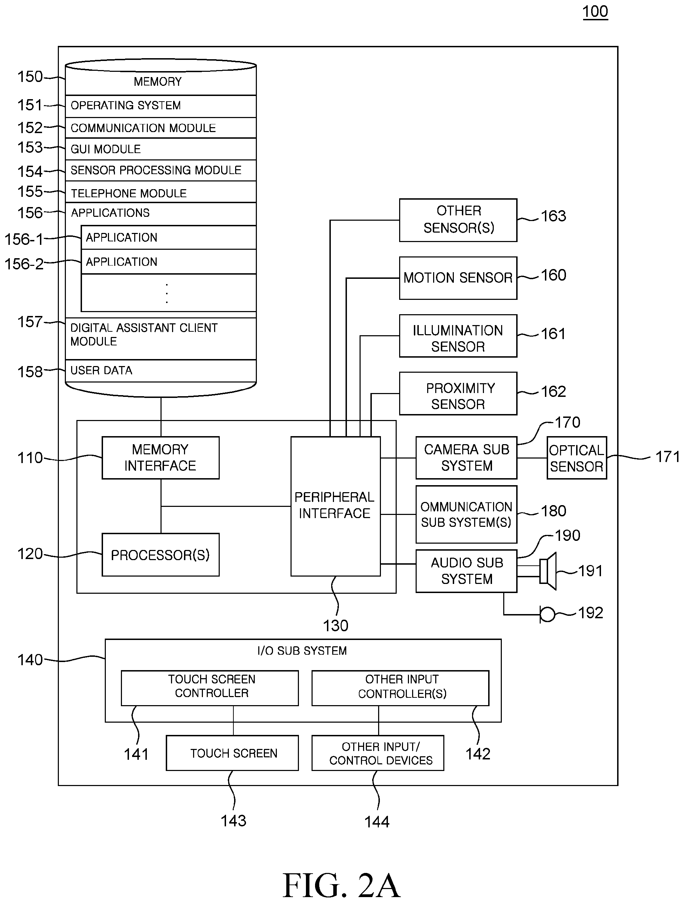

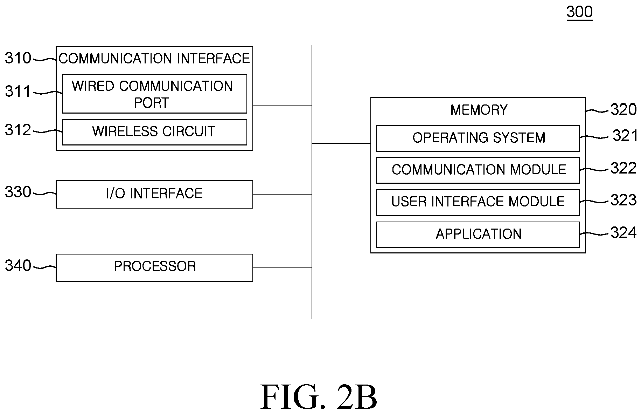

Hereinafter, referring to FIGS. 1, 2A and 2B, an information providing system for an ultrasound image view by using a device for providing information regarding an ultrasound image view according to an exemplary embodiment of the present invention and a device for providing information regarding an ultrasound image view will be described.

FIG. 1 illustrates an information providing system for an ultrasound image view using a device for providing information regarding an ultrasound image view according to an exemplary embodiment of the present invention. FIG. 2A exemplarily illustrates a configuration of a medical staff device which receives information regarding an ultrasound image view according to an exemplary embodiment of the present invention. FIG. 2B exemplarily illustrates a configuration of a device for providing information regarding an ultrasound image view according to an exemplary embodiment of the present invention.

First, referring to FIG. 1, an information providing system 1000 may be a system configured to provide information related to an ultrasound image view on the basis of an ultrasound image for an object. At this time, the information providing system 1000 may be configured by a medical staff device 100 which receives information related to an ultrasound image view, an ultrasound image diagnosis device 200 which provides an ultrasound image, and an information providing server 300 which generates information regarding the ultrasound image view on the basis of the received ultrasound image.

First, the medical staff device 100 is an electronic device which provides a user interface to indicate information related to the ultrasound image view and may include at least one of a smart phone, a tablet PC (personal computer), a laptop and/or a PC.

The medical staff device 100 may receive a prediction result related to an ultrasound image view for an object from the information providing server 300 and display the received result on a display unit to be described below.

The information providing server 300 may include a general purpose computer, a laptop and/or a data server which performs various computations to determine information related to the ultrasound image view on the basis of the ultrasound image provided from the ultrasound image diagnosis device 200, such as an ultrasound diagnosis device. At this time, the information providing server 300 may be a device which accesses a web server which provides a web page or a mobile web server which provides a mobile web site, but is not limited thereto.

To be more specific, the information providing server 300 may receive an ultrasound image from the ultrasound image diagnosis device 200 and classify the ultrasound image view in the received ultrasound image to provide information related to the ultrasound image view. At this time, the information providing server 300 may classify the ultrasound image view from the ultrasound image using a prediction model.

The information providing server 300 may provide a prediction result for the ultrasound image view to the medical staff device 100.

As described above, the information provided from the information providing server 300 may be provided to a web page through a web browser installed in the medical staff device 100 or provided as an application or a program. In various exemplary embodiments, the data may be provided to be included in a platform in a client-server environment.

Next, referring to FIGS. 2A and 2B, a component of the information providing server 300 of the present invention will be described in detail.

First, referring to FIG. 2A, the medical staff device 100 may include a memory interface 10, one or more processors 120, and a peripheral interface 130. Various components in the medical staff device 100 may be connected by one or more communication buses or signal lines.

The memory interface 110 is connected to a memory 150 to transmit various data to the processor 120. Here, the memory 150 may include at least one type of storing media of a flash memory type, a hard disk type, a multimedia card micro type, a card type memory (for example, an SD or XD memory), a RAM, an SRAM, a ROM, an EEPROM, a PROM, a network storage, a cloud, and block chain data.

In various exemplary embodiments, the memory 150 may store at least one or more of an operating system 151, a communication module 152, a graphic user interface module (GUI) 153, a sensor processing module 154, a telephone module 155, and an application module 156. Specifically, the operating system 151 may include a command for processing a basic system service and a command for performing hardware tasks. The communication module 152 may communicate with at least one of one or more other devices, computers, and servers. The graphic user interface module (GUI) 153 may process a graphic user interface. The sensor processing module 154 may process sensor-related functions (for example, process a received voice input using one or more microphones 192). The telephone module 155 may process telephone-related functions. The application module 156 may perform various functions of the user application, such as electronic messaging, web browsing, media processing, searching, imaging, or other processing functions. Further, the medical staff device 100 may store one or more software applications 156-1 and 156-2 (for example, an information providing application) related to any one type of service in the memory 150.

In various exemplary embodiments, the memory 150 may store a digital assistant client module 157 (hereinafter, simply referred to as a DA client module) and accordingly, may store commands for performing a function of the client of the digital assistant and various user data 158.

In the meantime, the DA client module 157 may acquire voice input, text input, touch input and/or gesture input of the user by means of various user interfaces (for example, I/O sub system 140) equipped in the medical staff device 100.

Further, the DA client module 157 may output audio-visual or tactile data. For example, the DA client module 157 may output data formed of a combination of at least two or more of voice, sound, a notice, a text message, a menu, a graphic, a video, an animation, and a vibration. Further, the DA client module 157 may communicate with a digital assistant server (not illustrated) using a communication sub system 180.

In various exemplary embodiments, the DA client module 157 may collect additional information about the surrounding environment of the medical staff device 100 from various sensors, sub systems, and peripheral devices to configure a context associated with the user input. For example, the DA client module 157 may infer the intention of the user by providing context information to the digital assistant server together with the user input. Here, the context information which may be accompanied by the user input may include sensor information, such as lighting, ambient noises, ambient temperature, an image of the surrounding environment, and a video. As another example, the context information may include a physical state (for example, a device alignment, a device position, a device temperature, a power level, a speed, an acceleration, a motion pattern, a cellular signal intensity, or the like) of the medical staff device 100. As still another example, the context information may include information related to a software state of the medical staff device 100 (for example, a process which is being executed in the medical staff device 100, installed program, past and present network activities, a background service, an error log, resource usage, or the like).

According to various exemplary embodiments, the memory 150 may include added or deleted commands and further, the medical staff device 100 may also include additional configurations other than the configurations illustrated in FIG. 2A or exclude some configurations.

The processor 120 may control an overall operation of the medical staff device 100 and run an application or a program stored in the memory 150 to perform various commands to implement an interface which provides information related to the ultrasound image view.

The processor 120 may correspond to an arithmetic device such as a central processing unit (CPU) or an application processor (AP). Further, the processor 120 may be implemented as an integrated chip (IC) such as a system of chip (SoC) in which various arithmetic devices, such as a neural processing unit (NPU), are integrated.

The peripheral interface 130 is connected to various sensors, sub systems, and peripheral devices to provide data to allow the medical staff device 100 to perform various functions. Here, when the medical staff device 100 performs any function, it is understood that the function is performed by a processor 120.

The peripheral interface 130 may receive data from a motion sensor 150, an illumination sensor (an optical sensor) 161, and a proximity sensor 162 and by doing this, the medical staff device 100 may perform alignment, light, and proximity sensing functions. As another example, the peripheral interface 130 may be provided with data from other sensors 163 (a positioning system-GPS receiver, a temperature sensor, or a biometric sensor) and by doing this, the medical staff device 100 may perform functions related to the other sensors 163.

In various exemplary embodiments, the medical staff device 100 may include a camera sub system 170 connected to the peripheral interface 130 and an optical sensor 171 connected thereto and by doing this, the medical staff device 100 may perform various photographing functions such as taking a picture or recording a video clip.

In various exemplary embodiments, the medical staff device 100 may include a communication sub system 180 connected to the peripheral interface 130. The communication sub system 180 is configured by one or more wired/wireless networks and may include various communication ports, a wireless frequency transceiver, and an optical transceiver.

In various exemplary embodiments, the medical staff device 100 includes an audio sub system 190 connected to the peripheral interface 130 and the audio sub system 190 includes one or more speakers 191 and one or more microphones 192 so that the medical staff device 100 may perform voice-operated functions, such as voice recognition, voice duplication, digital recording, and telephone functions.

In various exemplary embodiments, the medical staff device 100 may include an I/O sub system 140 connected to the peripheral interface 130. For example, the I/O sub system 140 may control the touch screen 143 included in the medical staff device 100 by means of a touch screen controller 141. For example, the touch screen controller 141 may use any one of a plurality of touch sensing techniques such as a capacitive type, a resistive type, an infrared type, a surface acoustic wave technology, or a proximity sensor array to detect contact and movement of the user or stopping of contact and movement. As another example, the I/O sub system 140 may control the other input/control device 144 included in the medical staff device 100 by means of other input controller(s) 142. As an example, other input controller(s) 142 may control one or more buttons, rocker switches, thumb-wheels, infrared ports, USB ports, and pointer devices such as a stylus.

Next, referring to FIG. 2B, the information providing server 300 may include a communication interface 310, a memory 320, an I/O interface 330, and a processor 340 and each configuration may communicate with each other via one or more communication buses or signal lines.

The communication interface 310 is connected to the medical staff device 100 and the ultrasound image diagnosis device 200 via a wired/wireless communication network to exchange data. For example, the communication interface 310 may receive an ultrasound image from the ultrasound image diagnosis device 200 and transmit determined information to the medical staff device 100.

In the meantime, the communication interface 310 which enables the transmission/reception of the data includes a wired communication port 311 and a wireless circuit 312 and the wired communication port 311 may include one or more wired interfaces, such as Ethernet, universal serial bus (USB), and fire wire. Further, the wireless circuit 312 may transmit and receive data with external devices by an RF signal or an optical signal. In addition, the wireless communication may use at least one of a plurality of communication standards, protocols, and technologies, such as GSM, EDGE, CDMA, TDMA, Bluetooth, Wi-Fi, VoIP, Wi-Max, or other arbitrary appropriate communication protocols.

The memory 320 may store various data used in the information providing server 300. For example, the memory 320 may store an ultrasound image or store an image view classification model and a second prediction model trained to divide an ultrasound image view, and further, a cervical canal region in the ultrasound image.

In various exemplary embodiments, the memory 320 may include a volatile or nonvolatile recording medium which may store various data, commands, and information. For example, the memory 320 may include at least one type of storage media of a flash memory type, a hard disk type, a multimedia card micro type, a card type memory (for example, an SD or XD memory), a RAM, an SRAM, a ROM, an EEPROM, a PROM, a network storage, a cloud, and block chain data.

In various exemplary embodiments, the memory 320 may store a configuration of at least one of an operating system 321, a communication module 322, a user interface module 323, and one or more applications 324.

The operating system 321 (for example, an embedded operating system, such as LINUX, UNIX, MAC OS, WINDOWS, VxWorks) may include various software components and drivers which control and manage a general system task (for example, memory management, storage device control, power management, or the like) and support communication between various hardware, firmware, and software components.

The communication module 323 may support communication with other devices through the communication interface 310. The communication module 320 may include various software components for processing data received by a wired communication port 311 or a wireless circuit 312 of the communication interface 310.

The user interface module 323 may receive a request or an input of the user from a keyboard, a touch screen, or a microphone via the I/O interface 330 and provide the user interface on the display.

The application 324 may include a program or a module configured to be executed by one or more processors 330. Here, the application for providing information related to an ultrasound image view may be implemented on a server farm.

The I/O interface 330 may connect at least one of input/output devices (not illustrated) of the information providing server 300, such as a display, a keyboard, a touch screen, and a microphone, to the user interface module 323. The I/O interface 330 may receive the user input (for example, voice input, keyboard input, or touch input) together with the user interface module 323 and process a command in accordance with the received input.

The processor 340 is connected to the communication interface 310, the memory 320, and the I/O interface 330 to control an overall operation of the information providing server 300 and may perform various commands to provide information through the application or the program stored in the memory 320.

The processor 340 may correspond to an arithmetic device such as a central processing unit (CPU) or an application processor (AP). Further, the processor 340 may be implemented as an integrated chip (IC) such as a system of chip (SoC) in which various arithmetic devices are integrated. Further, the processor 340 may include a module for calculating an artificial neural network model like a neural processing unit (NPU).

In various exemplary embodiments, the processor 340 may be configured to classify and provide the ultrasound image view in the ultrasound image using the prediction models. Optionally, the processor 340 may be configured to provide information regarding an ultrasound image view length.

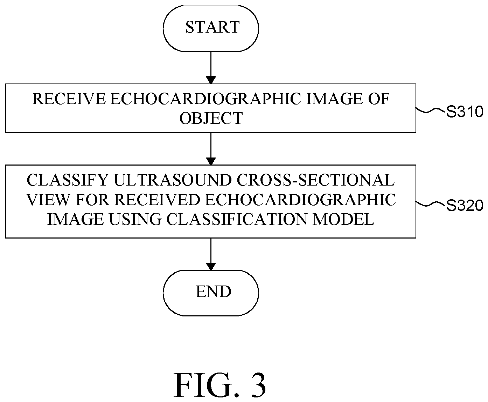

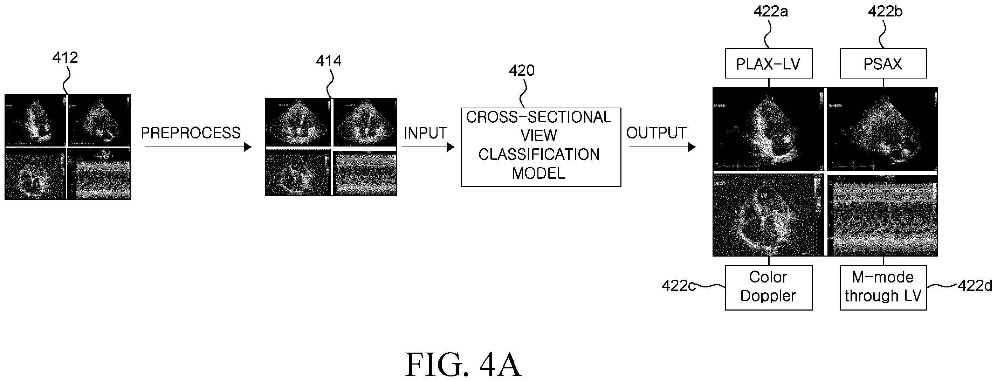

Hereinafter, a method for providing information regarding an ultrasound image view according to an exemplary embodiment of the present invention will be described in detail with reference to FIGS. 3, 4A to 4E.

FIG. 3 illustrates a procedure of a method for providing information regarding an ultrasound image view according to an exemplary embodiment of the present invention. FIGS. 4A to 4E exemplarily illustrate a procedure of a method for providing information regarding an ultrasound image view according to an exemplary embodiment of the present invention.

At this time, classification of views of the echocardiographic image will be described as an example, but the present invention is not limited thereto and an information providing method according to various exemplary embodiments of the present invention may be applied to ultrasound images of various objects which is capable of being ultrasound imaged.

First, referring to FIG. 3, the information providing procedure according to an exemplary embodiment of the present invention is as follows: First, an ultrasound image for an object is received in step S310. Next, an image view for each ultrasound mode is classified by an image view classification model in step S320.

To be more specific, in the step S310 of receiving an ultrasound image, an ultrasound image for a target area, that is, a heart area, may be received.

According to a feature of the present invention, in the step S310 of receiving an ultrasound image, an ultrasound image of a DICOM format may be received. At this time, the ultrasound image of a DICOM format may include metadata, such as a set ultrasound mode.

Next, the step S320 of classifying respective ultrasound image views is performed.

According to the feature of the present invention, in the step S320 of classifying respective ultrasound image views, image views for an M-mode, a B-mode, and a Doppler mode may be classified by the image view classification model, respectively. However, the present invention is not limited thereto and the image view in a non-classified mode which is different from the ultrasound mode may be further classified.

For example, referring to FIG. 4A together, in the step S320 of classifying respective ultrasound image views, the ultrasound image 412 is preprocessed and the preprocessed ultrasound image 414 is input to the image view classification model 420. At this time, the image view classification model may be a model trained to classify an image view for an M-mode, an image view for a B-mode, and an image view for a Doppler mode with an ultrasound image for training as an input. However, the present invention is not limited thereto and the image view classification model may be a model trained to further classify an image view of a non-classified mode, such as "color Doppler" for the input ultrasound image.

Consequently, classification results 422a, 422b, 422c, and 422d for each of the plurality of ultrasound modes are output from the image view classification model 420.

That is, even though the received ultrasound image 412 is an image captured in a plurality of ultrasound modes, images corresponding to respective image views are classified to be provided by the image view classification model.

In the meantime, according to another feature of the present invention, prior to the step S320 of classifying the respective ultrasound image views, the received ultrasound image may be preprocessed.

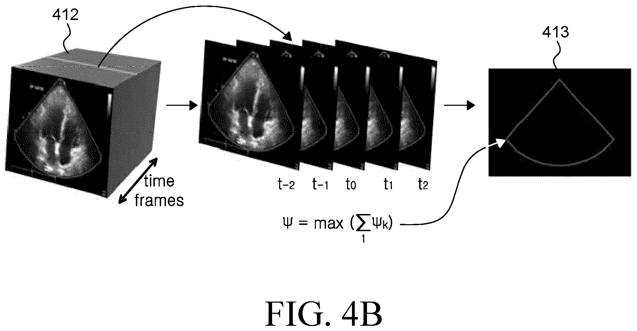

For example, referring to FIG. 4B together, in the step of performing preprocessing, a boundary which forms a fan-shaped echocardiographic area may be maximized for an ultrasound image 412 of a moving image, having a multi-frame, such as a B-mode image. As a result, an image 413 in which the boundary area is maximized may be acquired. However, the present invention is not limited thereto and in the step of performing preprocessing, a boundary which forms an echocardiographic area may be maximized for a single frame, that is, an ultrasound image of a still cut.

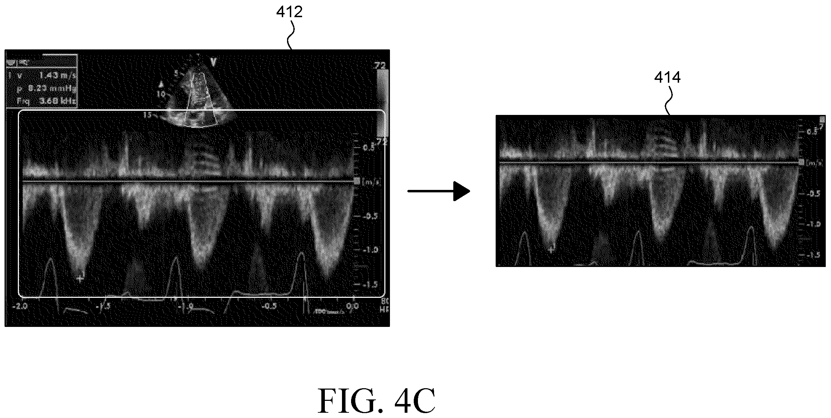

Further referring to FIG. 4C, in the step of performing preprocessing, in an ultrasound image 412 including a rectangular echocardiographic area, such as the M-mode or the Doppler mode, only a corresponding area may be cropped on the basis of a coordinate value of a DICOM header. As a result, a preprocessed ultrasound image 414 may be acquired.

That is, referring to FIG. 4A again, the ultrasound image 414 preprocessed according to various exemplary embodiments may be input to the image view classification model 420.

In the meantime, in various exemplary embodiments of the present invention, after the step S320 of classifying the respective ultrasound image views, the respective ultrasound image views may be verified.

According to the feature of the present invention, in the verifying step, an image view classified based on metadata which is obtained from the image of DICOM format may be verified and corrected.

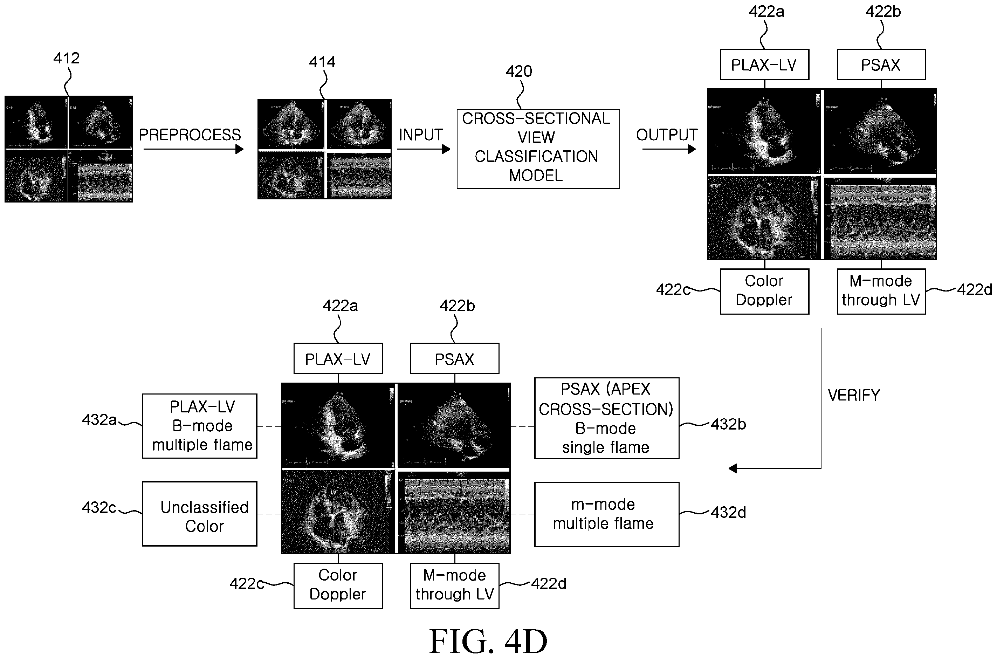

For example, referring to FIG. 4D together, metadata-based verification may be performed on the image view classification results 422a, 422b, 422c, and 422d for each of the plurality of ultrasound modes, classified by the image view classification model 420.

To be more specific, in the step of performing verification, it may be determined whether the image view image is a still cut image or a moving image and whether it is a color image or a monochromic image, with respect to the image view classification results 422a, 422b, 422c, and 422d.

Moreover, in the step of performing verification, the verification is performed on the image view classification results 422a, 422b, 422c, and 422d on the basis of the DICOM tagging information, that is, a previously stored ultrasound mode of the metadata and if it is different from the previously stored ultrasound mode, the classification result may be corrected.

In a specific exemplary embodiment, when one ultrasound image view of the B-mode of "PSAX" 422b is classified in the ultrasound image 414 input by the image view classification model 420, the classification result of "PSAX" 422b may be corrected to "PSAX (apex cross-section) on the basis of the ultrasound mode previously stored in the metadata, by the step of performing verification. That is, as the classification result of the image view classification model 420, the ultrasound mode may be finally determined by the verification.

That is, as the result of the step of performing verification, the verification results 432a, 432b, 432c, and 432d are provided so that not only the image view for the ultrasound image, but also more various information may be provided.

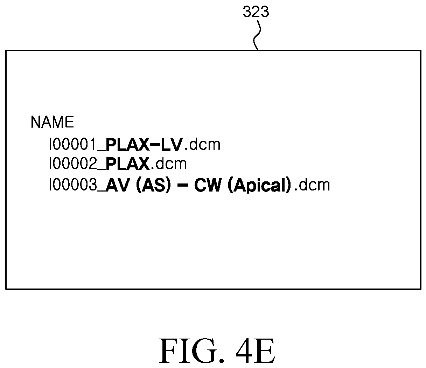

Further referring to FIG. 4E, in various exemplary embodiments, a file name in which an image view classified from the ultrasound image is described may be output through the user interface module 323 described above in FIG. 2B.

Therefore, according to the information providing method according to various exemplary embodiments of the present invention, the medical staff may easily identify the image view without a confirmation procedure for the acquired ultrasound image to more quickly perform the ultrasound analysis step and more accurately make a decision and establish a treatment plan.

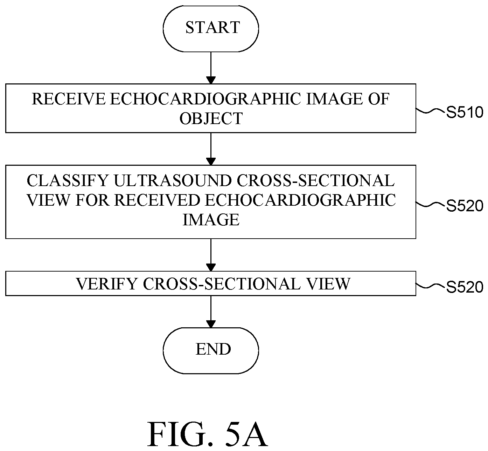

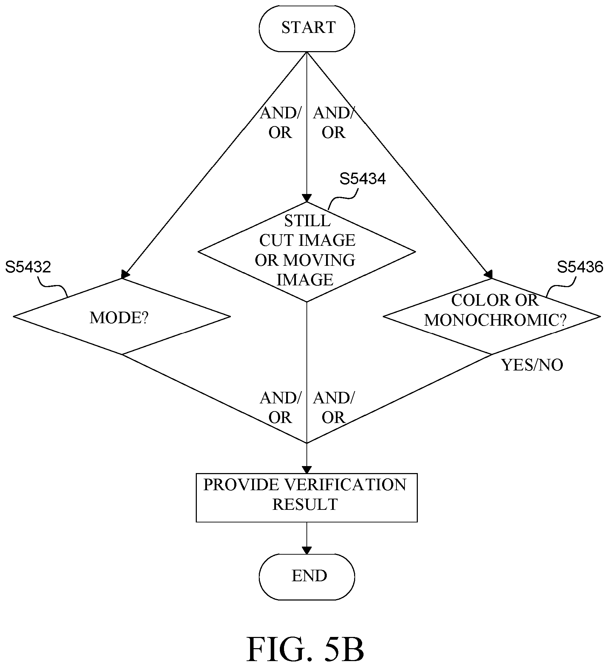

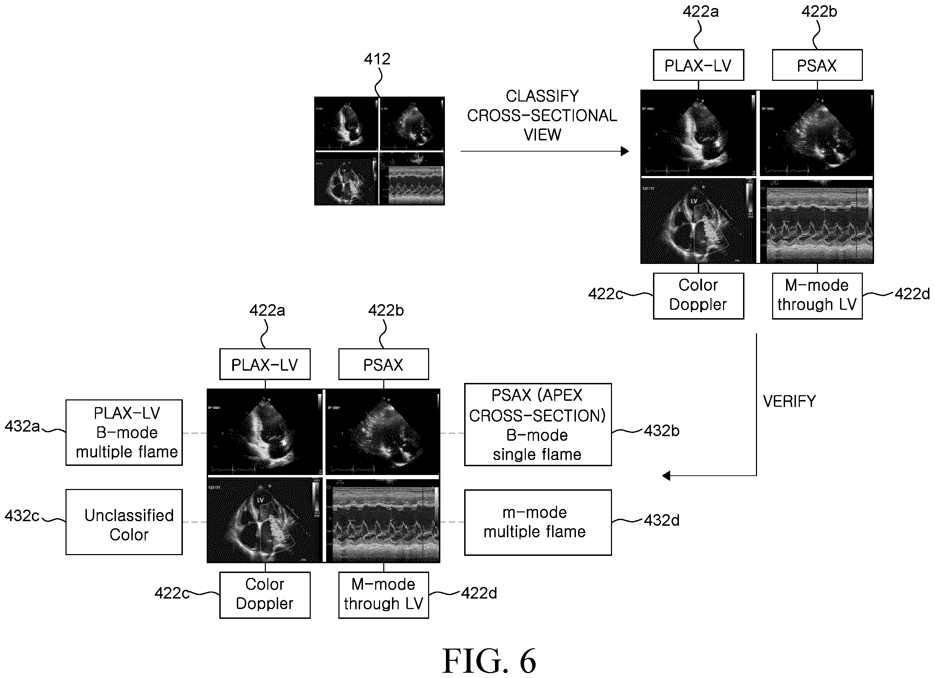

Hereinafter, a method for providing information regarding an ultrasound image view according to another exemplary embodiment of the present invention will be described with reference to FIGS. 5A, 5B, and 6.

FIGS. 5A and 5B illustrate a procedure of a method for providing information regarding an ultrasound image view according to another exemplary embodiment of the present invention. FIG. 6 exemplarily illustrates a procedure of a method for providing information regarding an ultrasound image view according to another exemplary embodiment of the present invention.

First, in order to provide information regarding an ultrasound image view according to another exemplary embodiment of the present invention, an ultrasound image of an object is received in step S510, image views for received respective ultrasound images are classified in step S520, and a classified image view is verified in step S530.

To be more specific, when an echocardiographic image, specifically, an ultrasound image of a DICOM format is obtainable from the ultrasound diagnostic device, by means of the step S510 of receiving an ultrasound image, in the step S520 of classifying respective image views for the ultrasound image, images corresponding to the plurality of image views are classified.

Next, referring to FIG. 5B together, in the step S530 of verifying the image view, it is determined what the ultrasound image for the classified image view is in step S5432, optionally, it is determined whether an image corresponding to the classified image view is a still cut or a moving image in step S5434, and optionally, it is determined whether an image corresponding to the classified image view is a color image or a monochromic image in step S5436. At this time, according to the feature of the present invention, in the step S530 of verifying an image view, the image view may be verified on the basis of metadata tagged to the DICOM image.

That is, in the step S530 of verifying an image view, not only the classified image view is verified, but also various information regarding the image may be determined. At this time, as a header value in the DICIOM, information regarding an image view type may be included.

For example, referring to FIG. 6 together, metadata-based verification may be performed on the image view classification results 422a, 422b, 422c, and 422d for each of the plurality of ultrasound modes, in the step S530 of verifying an image view.

To be more specific, in the step S530 of verifying an image view, it may be determined whether the image view image is a still cut image or a moving image and whether it is a color image or a monochromic image, with respect to the image view classification results 422a, 422b, 422c, and 422d.

At this time, the B-mode image may be a moving image format and the M-mode and the Doppler mode may be a still cut image format. Therefore, after distinguishing whether it is a still cut image or a moving image, the image may be classified in more detail.

Moreover, in the step S530 of verifying an image view, the verification may be performed on the image view classification results 422a, 422b, 422c, and 422d on the basis of the DICOM tagging information regarding the ultrasound mode, that is, a previously stored ultrasound mode of the metadata and if it is different from the previously stored ultrasound mode, the classification result may be corrected.

In a specific exemplary embodiment, when one ultrasound image view of the B-mode of "PSAX" 422b is classified, the classification result of "PSAX" 422b may be corrected to "PSAX (apex cross-section) on the basis of the ultrasound mode previously stored in the metadata (or tagging information in the DICOM image), by the step of performing verification. That is, the image view classification results 422a, 422b, 422c, and 422d are verified to finally determine the ultrasound mode.

That is, as the result of the step of performing verification, the verification results 432a, 432b, 432c, and 432d are provided so that not only the image view for the ultrasound image, but also more various information may be provided.

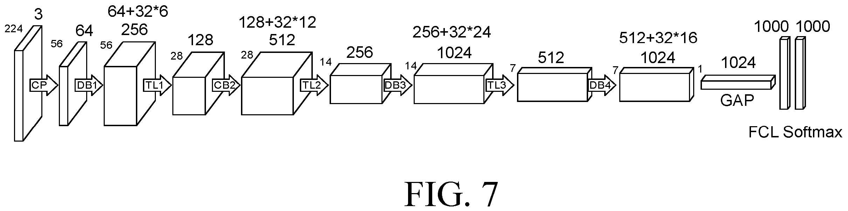

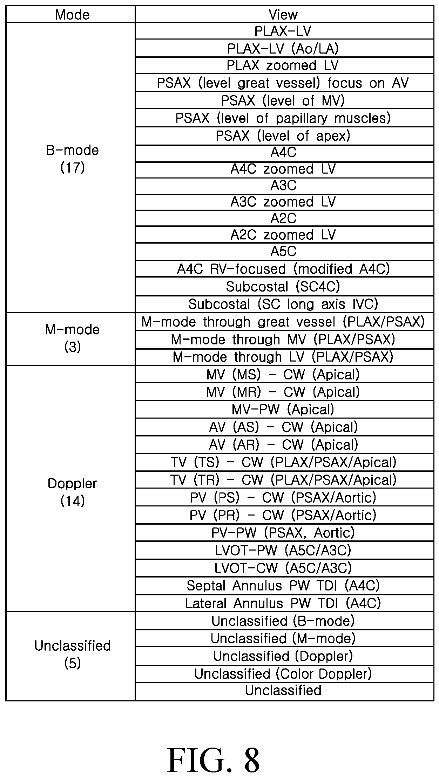

Hereinafter, a structural characteristic and learning of an information prediction model related to an ultrasound image view used in various exemplary embodiments of the present invention will be described with reference to FIGS. 7 and 8.

FIG. 7 exemplarily illustrates a structure of an image view classification model which is used for a method for providing information regarding an ultrasound image view according to an exemplary embodiment of the present invention. FIG. 8 exemplarily illustrates learning data of an image view classification model which is used for an information providing method according to various exemplary embodiments of the present invention.

First, referring to FIG. 7, an image view classification model used for various exemplary embodiments of the present invention may have a structure of DeseNet 121. Specifically, the DenseNet 121-based image view classification model may be configured by a plurality of blocks in which computation is performed.

To be more specific, the image view classification model is formed by a CP block configured by a convolution layer which performs computation on the input ultrasound image and a pulling layer, a DB block which is configured by a plurality of dense layers to add a feature map as much as a predetermined growth rate whenever it passes through each dense layer, and a TL layer of a transfer layer present between the DB blocks. Moreover, the multifaceted classification model may include an output layer of a fully connected layer and a softmax layer which output the classified ultrasound image view.

According to the feature of the present invention, the number of nodes of the output layer may correspond to the number of ultrasound image views.

For example, further referring to FIG. 8, the ultrasound image view classification model used for various exemplary embodiments of the present invention may be trained to classify image views corresponding to respective images for the ultrasound images labeled with a total of 39 image views including 17 image views corresponding to B-modes, three image views corresponding to M-modes, 14 image views corresponding to Doppler modes, and five image views corresponding to non-classified modes.

At this time, the number of nodes of the output layer of the image view classification model may be equal to 39 which is the number of image views to be classified, set in the learning step.

However, the learning data of the image view classification model is not limited to those described above. For example, ultrasound images tagged with image views of a combination of the B-mode and the M-mode, a combination of the M-mode and the Doppler mode, or a combination of the B-mode and the Doppler mode may be set as learning data.

However, the structure of the image view classification model is not limited to those described above.

Evaluation: Evaluation of image view classification model used for various exemplary embodiments of present invention

Hereinafter, performances of an image view classification model used for various exemplary embodiments of the present invention and models based on the related art are compared and described.

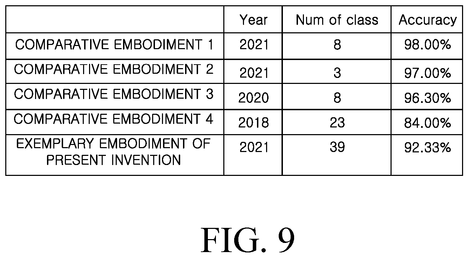

FIG. 9 illustrates an evaluation result of an image view classification model which is used for an information providing method according to various exemplary embodiments.

To be more specific, referring to FIG. 9, classification using a classification model according to an exemplary embodiment of the present invention represents a high accuracy of 92.33% even though the number of ultrasound image view classes for classification is 39.

In the meantime, classification results of Comparative Embodiment 1 (Andreas Ø et al.), Comparative Embodiment 2 (Ivar M. Salte, MD et al.), and Comparative Embodiment 3 (Ahmed I. Shanin et al.) show high accuracy of 98%, 97%, and 96.3%, respectively. However, the classification classes of Comparative Embodiments are 8, 3, and 8 and the classification accuracies are obtained from 8 or fewer image views of a single mode.

The machine learning-based classification result of Comparative Embodiment 4 (Jeffrey Zhang et al.) is obtained by classifying 23 image view classes, but a classification accuracy is 84%, which is lower than the accuracy (92.33%) of the classification according to the information providing method according to the exemplary embodiment of the present invention.

This result may mean that the image view classification model used for various exemplary embodiments of the present invention may classify more image views, but have more excellent classification performance than the related art.

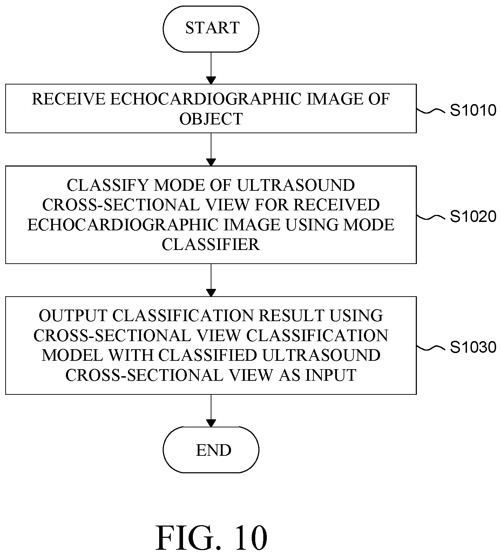

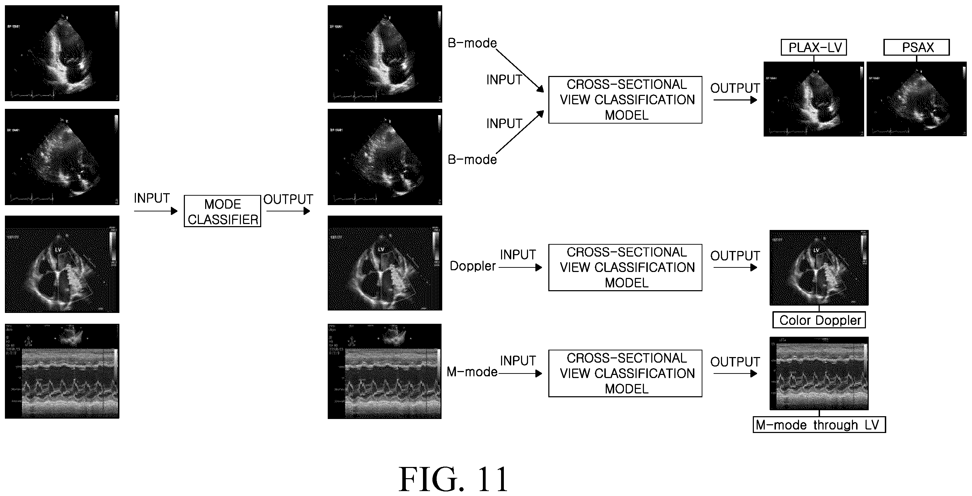

Hereinafter, a method for providing information regarding an ultrasound image view according to various exemplary embodiments of the present invention will be described in detail with reference to FIGS. 10 and 11.

FIG. 10 illustrates a procedure of a method for providing information regarding an ultrasound image view according to an exemplary embodiment of the present invention.

FIG. 11 exemplarily illustrates a procedure of a method for providing information regarding an ultrasound image view according to an exemplary embodiment of the present invention.

At this time, classification of views of the echocardiographic image will be described as an example, but the present invention is not limited thereto and an information providing method according to various exemplary embodiments of the present invention may be applied to ultrasound images of various objects which is capable of being ultrasound imaged.

First, referring to FIG. 10, the information providing procedure according to an exemplary embodiment of the present invention is as follows: First, an ultrasound image for an object is received in step S1010. Next, a mode of an ultrasound cross-sectional view of the received echocardiographic image is classified using a mode classifier in step S1020.

Next, a classification result is output using a cross-sectional view classification model using the classified ultrasound cross-sectional view as an input in step S1030.

To be more specific, in the step S 1010 of receiving an ultrasound image, an ultrasound image for a target area, that is, a heart area, may be received.

Next, a step S1020 of classifying a mode of an ultrasound cross-sectional view of the received echocardiographic image using a mode classifier is performed.

According to the feature of the present invention, image views for the M-mode, the B-mode, and the Doppler mode may be classified on the basis of metadata which can be acquired from an image of DICOM format in the step S1020 of classifying a mode of an ultrasound cross-sectional view using a mode classifier, or image views for the M-mode, the B-mode, and the Doppler mode may be classified using the artificial intelligence model. However, the present invention is not limited thereto and the image view in a non-classified mode which is different from the ultrasound mode may be further classified.

For example, referring to FIG. 11 together, in the step S1020 of classifying respective ultrasound image views, the ultrasound image is input to the mode classifier to classy the modes and the classification result for every mode is output using the cross-sectional view classification model using the mode-classified ultrasound image as an input.

At this time, as an example, the mode classifier may classify the M-mode, the B-mode, and the Doppler mode on the basis of metadata which can be acquired from the image of the DICOM format or using the artificial intelligence model.

The results for respective modes are output using the cross-sectional view classification model using the ultrasound image classified by the mode classifier as an input. At this time, the cross-sectional view classification model may be any one of a B-mode cross-sectional view classification model, an M-mode cross-sectional view classification model, or a Doppler mode cross-sectional view classification model, but is not limited thereto.

That is, the received ultrasound image may be classified and provided for each of the M-mode, the B-mode, and the Doppler mode, on the basis of the metadata which can be acquired from the image of DICOM format or using the mode classifier using the artificial intelligence model. The classification result is output and provided using a classification model corresponding to respective classified modes, among the B-mode cross-sectional view classification model, the M-mode cross-sectional view classification model, or the Doppler mode cross-sectional view classification model, with the classified ultrasound image as an input.

In the meantime, according to another feature of the present invention, prior to the step S1120 of classifying the respective ultrasound image view, the received ultrasound image may be preprocessed, which is the same as described above with reference to FIG. 4B.

Therefore, according to the information providing method according to various exemplary embodiments of the present invention, the medical staff may easily identify the image view for the obtained ultrasound image to more quickly perform the ultrasound analysis step and more accurately make a decision and establish a treatment plan.

Although the exemplary embodiments of the present invention have been described in detail with reference to the accompanying drawings, the present invention is not limited thereto and may be embodied in many different forms without departing from the technical concept of the present invention. Accordingly, the various exemplary embodiments disclosed herein are not intended to limit the technical spirit of the present invention but describe with the true scope and spirit being indicated by the following claims and the scope of the technical spirit of the present invention is not limited to the exemplary embodiments. Thus, it is to be appreciated that exemplary embodiments described above are intended to be illustrative in every sense, and not restrictive. The protective scope of the present invention should be construed based on the following claims, and all the technical concepts in the equivalent scope thereof should be construed as falling within the scope of the present invention.

## Claims

1. A method for providing information regarding an ultrasound image view, which is implemented by a processor, comprising:
receiving an ultrasound image of an object; and
classifying respective image views on the basis of the received ultrasound image, by using an image view classification model trained to classify a plurality of image views by using the ultrasound image as an input and output respective image views,
wherein the respective image views indicate the ultrasound image views in a plurality of different ultrasound modes.

2. The method for providing information regarding an ultrasound image view according to claim 1, wherein the plurality of ultrasound modes includes an M-mode and a B-mode, the ultrasound image is an echocardiographic image, and
the classifying respective image views includes determining at least one M-mode image view, among an M-mode (PLAX/PSAX) through a blood vessel, an M-mode (PLAX/PSAX) through MV, and an M-mode (PLAX/PSAX) through LV and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the echocardiographic image, using the image view classification model.

3. The method for providing information regarding an ultrasound image view according to claim 1, wherein the plurality of ultrasound modes includes a Doppler mode and a B-mode, the ultrasound image is an echocardiographic image, and
the classifying respective image views includes determining at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the echocardiographic image, using the image view classification model.

4. The method for providing information regarding an ultrasound image view according to claim 1, wherein the plurality of ultrasound modes includes a Doppler mode, an M-mode, and a B-mode and the ultrasound image is an echocardiographic image, and
the classifying respective image views includes determining at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), at least one M-mode image view, among an M-mode (PLAX/PSAX) through a great artery, an M-mode (PLAX/PSAX) through MV, and an M-mode (PLAX/PSAX) through LV, and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the echocardiographic image, using the image view classification model.

5. The method for providing information regarding an ultrasound image view according to claim 1, wherein the plurality of ultrasound modes includes at least one of an M-mode, a Doppler mode, and a B-mode and a non-classified mode defined as an ultrasound image view which is different from the M-mode, the Doppler mode, and the B-mode.

6. The method for providing information regarding an ultrasound image view according to claim 1, wherein the ultrasound image is an echocardiographic image, and
the classifying respective image views includes:
classifying 4-chamber, 2-chamber, long axis, base, mid, and apex image views for the echocardiographic image, using the image view classification model.

7. The method for providing information regarding an ultrasound image view according to claim 1, further comprising:
after the classifying respective image views, verifying the respective image views.

8. The method for providing information regarding an ultrasound image view according to claim 7, wherein the verifying the respective image views includes:
determining whether the ultrasound image is a still cut image or a moving image, on the basis of the number of frames of the respective image views.

9. The method for providing information regarding an ultrasound image view according to claim 7, wherein the verifying the respective image views includes:
determining whether the respective image views are color images or monochromic images.

10. The method for providing information regarding an ultrasound image view according to claim 7, wherein the ultrasound image is an image of digital imaging and communications in medicine (DICOM) format displaying metadata including tagging information regarding the ultrasound mode of the ultrasound image and
verifying the respective image views includes:
verifying the respective views on the basis of the metadata, and
after the verifying, further includes: correcting a classification result of the respective image views when the classified image view is different from the tagging information.

11. The method for providing information regarding an ultrasound image view according to claim 1, wherein the image view classification model has output nodes corresponding to the number of the plurality of image views according to the plurality of ultrasound modes.

12. A device for providing information regarding an ultrasound image view, comprising:
a communication unit configured to receive an ultrasound image of an object; and
a processor operably connected to the communication unit,
wherein the processor is configured to classify respective image views on the basis of the received ultrasound image, by using an image view classification model trained to classify a plurality of image views by using the ultrasound image as an input and output respective image views, and
the respective image views indicate the ultrasound image views in a plurality of different ultrasound modes.

13. The device for providing information regarding an ultrasound image view according to claim 12, wherein the plurality of ultrasound modes includes an M-mode and a B-mode and the ultrasound image is an echocardiographic image, and
the processor is configured to determine at least one M-mode image view, among an M-mode (PLAX/PSAX) through a great artery, an M-mode (PLAX/PSAX) through MV, and an M-mode (PLAX/PSAX) through LV and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the echocardiographic image, using the image view classification model.

14. The device for providing information regarding an ultrasound image view according to claim 12, wherein the plurality of ultrasound modes includes a Doppler mode and a B-mode and the ultrasound image is an echocardiographic image, and
the processor is configured to determine at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C) and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the echocardiographic image, using the image view classification model.

15. The device for providing information regarding an ultrasound image view according to claim 12, wherein the plurality of ultrasound modes includes a Doppler mode, an M-mode, and a B-mode and the ultrasound image is an echocardiographic image, and
the processor is configured to determine at least one Doppler mode image view, among MV (MS) - CW (Apical), MV (MR) - CW (Apical), MV-PW (Apical), AV (AS) - CW (Apical), AV (AR) - CW (Apical), TV (TS) - CW (PLAX/PSAX/Apical), TV (TR) - CW (PLAX/PSAX/Apical), PV (PS) - CW (PSAX/Aortic), PV (PR) - CW (PSAX/Aortic), PV-PW (PSAX, Aortic), LVOT-PW (A5C/A3C), LVOT-CW (A5C/A3C), septal annulus PW TDI (A4C), and lateral annulus PW TDI (A4C), at least one M-mode image view, among an M-mode (PLAX/PSAX) through a great artery, an M-mode (PLAX/PSAX) through MV, and an M-mode (PLAX/PSAX) through LV, and at least one B-mode image view, among PLAX-LV, PLAX-LV (Ao/LA), LV zoomed PLAX, AV focused PSAX (great artery cross-section), PSAX (MV cross-section), PSAX (papillary muscle cross-section), PSAX (apex cross-section), A4C, A4C, A3C, LV zoomed A3C, A2C, LV zoomed A2C, A5C, RV-focused A4C (deformed A4C), subcostal (SC4C), and subcostal (SC long axis IVC), for the echocardiographic image, using the image view classification model.

16. The device for providing information regarding an ultrasound image view according to claim 12, wherein the plurality of ultrasound modes includes at least one of an M-mode, a Doppler mode, and a B-mode and a non-classified mode defined as an ultrasound image view which is different from the M-mode, the Doppler mode, and the B-mode.

17. The device for providing information regarding an ultrasound image view according to claim 12, wherein the ultrasound image is an echocardiographic image, and
the processor is configured to classify 4-chamber, 2-chamber, long axis, base, mid, and apex image views for the echocardiographic image, using the image view classification model.

18. The device for providing information regarding an ultrasound image view according to claim 12, wherein the processor is further configured to verify the respective image views.

19. The device for providing information regarding an ultrasound image view according to claim 18, wherein the processor is further configured to determine whether the ultrasound image is a still cut image or a moving image, on the basis of the number of frames of the respective image views.

20. The device for providing information regarding an ultrasound image view according to claim 18, wherein the processor is further configured to determine whether the respective image views are color images or monochromic images.

21. The device for providing information regarding an ultrasound image view according to claim 18, wherein the ultrasound image is an image of digital imaging and communications in medicine (DICOM) format displaying metadata including tagging information regarding the ultrasound mode of the ultrasound image and
the processor is further configured to verify the respective image views on the basis of the metadata and correct a classification result of the respective image views when the respective classified image views are different from the tagging information.

22. The device for providing information regarding an ultrasound image view according to claim 12, wherein the image view classification model has output nodes corresponding to the number of the plurality of image views according to the plurality of ultrasound modes.

23. A method for providing information regarding an ultrasound image view, which is implemented by a processor, comprising:
receiving an ultrasound image of an object; and
classifying a mode of an ultrasound cross-sectional view of the received ultrasound image using a mode classifier; and
outputting and providing a classification result using a classification model with the classified ultrasound cross-sectional view as an input.

24. The method for providing information regarding an ultrasound image view according to claim 23, wherein the mode of the ultrasound cross-sectional view includes a Doppler mode, an M-mode, and a B-mode and the ultrasound image is an echocardiographic image.

25. The method for providing information regarding an ultrasound image view according to claim 23, wherein the classifying a mode of an ultrasound cross-sectional view of the received ultrasound image using a mode classifier includes:
classifying an image view on the basis of metadata which is obtainable from an image of DICOM format.

26. The method for providing information regarding an ultrasound image view according to claim 23, wherein the classification model is any one of a B-mode classification model, an M-mode classification model, or a Doppler mode classification model.
